Europäisches Patentamt

European Patent Office

Office européen des brevets

(19)

(11) Publication number: **0 236 040**
**A2**

(12) # EUROPEAN PATENT APPLICATION

(21) Application number: **87301567.1**

(22) Date of filing: **24.02.87**

(51) Int. Cl.⁴: **C 12 N 9/72**
**C 12 N 15/00**

(30) Priority: **26.02.86 US 833179   19.02.87 US 12023**

(43) Date of publication of application:
**09.09.87   Bulletin   87/37**

(84) Designated Contracting States:
**AT BE CH DE ES FR GB GR IT LI LU NL SE**

(71) Applicant: **COLLABORATIVE RESEARCH INC.**
**Two Oak Park**
**Bedford Massachusetts 01730  (US)**

(72) Inventor: **Vovis, Gerald F.**
**360 Bacon Street**
**Waltham Massachusetts 02154  (US)**

**Mao, Jen-I**
**213 Follen Road**
**Lexington Massachusetts 02173  (US)**

(74) Representative: **Shipley, Warwick Grenville Michael et al**
**VENNER, SHIPLEY & CO. 368 City Road**
**London EC1V 2QA  (GB)**

(54) Amino acid modified prourokinase and method of preparation.

(57) A structurally modified single chain prourokinase having a lysine cleavage site removed to resist change to the two chain form. Amino acid modified form of prourokinase has high fibrin selectivity and resistance to cleavage to the two chain form to an extent greater than its original unmodified form.

**Description**

AMINO ACID MODIFIED PROUROKINASE AND METHOD OF PREPARATION

RELATED APPLICATION

This application is a continuation-in-part of United States Patent Application Serial No. 06/833179 filed February 26, 1986.

BACKGROUND OF THE INVENTION

Since at least the 1940s, human urine has been known to contain an activity capable of converting plasminogen, the proteolytic zymogen present in plasma, into the enzyme plasmin. This plasminogen activator activity was named urokinase. Different molecular forms of this urinary, plasminogen activator activity have since been described. However, all of these forms are believed to be derived from substantially the same precursor molecule, prourokinase.

Prourokinase was so named because when it was discovered it was thought to be the zymogen form of the molecule. It has since been shown to have biological activity (Lijnen, H.R., Zamarron, D., Blaber, M., Winkler, M.E., and Collen, D. (1986) J. Biol. Chem. 261: 1253-1258 and Collen, D., Zamarron, D., Lijnen, H.R., and Hoylaerts, M. (1986) J. Biol. Chem. 261: 1259-1266). As a result, the acronym SCUPA (single chain urinary plasminogen activator) has been proposed as a better name for this form of the molecule. Prourokinase consists of a single polypeptide chain 411 amino acid residues long. The two chain form, called high molecular weight urokinase (HMW), consists of two polypeptide chains held together in a single molecule by disulfide bond(s). Prourokinase is converted to HMW urokinase by the proteolytic cleavage of the peptide bond between lysine[158] and isoleucine[159]. Low molecular weight (LMW) urokinase is a single polypeptide chain and consists of amino acid residues 159 through 411 of the prourokinase molecule. All three forms are activators of plasminogen.

Plasminogen activators have received significant attention because of their use as thrombolytic agents. Of the three forms of urokinase described above, only prourokinase is considered to be a thrombolytic agent for the treatment of myocardial infarction, pulmonary embolism, and deep vein thrombosis. Neither HMW nor LMW urokinase exhibit any particular substrate specificity. Hence, they do not cause significant clot lysis without also producing significant systemic fibrinolytic activation. On the other hand, prourokinase shows fibrin selectivity. That is, prourokinase lyses clots without significant systemic fibrinolytic activation occurring in most patients.

To be therapeutically desirable, however, prourokinase is preferably free of any contaminating HMW or LMW urokinase. Isolation of prourokinase from natural sources, urine or the conditioned medium of human cells cultured in vitro, often involves the removal of HMW and/or LMW urokinase produced from prourokinase by proteases present in urine or culture medium. Prourokinase prodced from host cells as the result of recombinant DNA manipulations also contains HMW and/or LMW urokinase produced as the result of proteases present inside or in the medium outside these hosts. In addition, prourokinase can be converted into HMW and LMW urokinase in vivo after therapeutic administration. This conversion can occur before the prourokinase molecule reaches the thrombii, the site for which it alone amongst the biologically active forms of urokinase shows enhanced specificity. Thus, HMW and/or LMW urokinase present in prourokinase preparations or created in vivo before the administered prorokinase reaches the thrombii reduces the therapeutic specificity of the prourokinase treatment.

Consequently, significant therapeutic benefit is to be gained by having a prourokinase molecule unable or highly resistant to conversion to HMW and LMW urokinase. Such a molecule could have little or no systemic fibrinolytic activation associated with its thereapeutic administration.

Currently there is interest in site-directed mutagenic approaches to the study of fundamental structure-function problem in proteins. See Ackers, G. K. and Smith, F. R. (1985) Ann. Rev. Biochem. 54: 597-629. The ability to manipulate some protein structures by altering individual amino acid residues at desired locations through genetic engineering is known.

SUMMARY OF THE INVENTION

It is an object of this present invention to provide modified prourokinase having a stable single polypeptide chain.

Another object of this invention is to provide a modified prourokinase having a single polypeptide chain in accordance with the preceding object with the lysine amino acid residue normally at position 158 being changed to an amino acid residue other than lysine.

Still another object of this invention is to provide means and methods of modifying prourokinase in accordance with the preceding objects.

Still another object to the invention is to provide the means and methods for producing full length biologically active prourokinase molecules from DNA expression vehicles harboring gene sequences encoding the modified prourokinase in expressible form.

A further object of the present invention is to provide organisms transformed with the expression vehicles in accordance with the preceding object capable of directing production of prourokinase.

Amino acid modified prourokinase, according to the present invention, is provided from recombinant DNA techniques with an amino acid modification such that cleavage of the lysine[158] - isoleucine[159] peptide bond does not readily occur and the polypeptide is maintained as a single-chain not readily susceptible to cleavage to two-chain.

A method is provided for increasing the therapeutic specificity of single-chain prorokinase without substantially decreasing fibrinolytic activity, which comprises replacing the lysine residue at position l58 of the amino acid sequence of prourokinase.

It is a feature of this invention that prourokinase having a molecular weight of about 50,000 in the form of a single polypeptide chain can be particularly effective for use in dissolving blood clots, in the living human body, if it can be maintained in that form for a longer time period than previously known prourokinase. The single chain form can have high fibrin affinity and thus is not believed to cause general bleeding in the body but rather can be localized to the site of the blood clot directly.

It is a further feature of this invention that modification of the prourokinase coding sequence by site-directed mutagenesis or other means, results in stabilization of the single chain so as to maintain it in the single chain polypeptide form and thus increase its value for therapeutic use over that of naturally occurring prourokinase .

## BRIEF DESCRIPTION OF THE DRAWINGS

Table l depicts the nucleotide sequence of prourokinase and the amino acid sequence deduced. The amino acids are numbered from l to 4ll. The arrow indicates the start of the initial isolated clone;

Table 2 is a schematic representation of probes useful in this invention;

Table 3 shows the plasmid, pSV2, used for the expression of prourokinase in mouse NIH 3T3 cells; and

Table 4 illustrates the construction of the plasmid containing prourokinase with an alanine at amino acid position l58.

Table 5 shows the gel electrophoreris results of natural prourokinase and prourokinase that has a methionine substituted at position l58 and a serine substituted at position l60 lys[158] - ser[160] prourokinase following treatment with human plasmin. Lane l-300 microliters of prourokinase; Lane 2-300 microliters of prourokinase previously treated with ll microliters (0.0l5 mg/ml) plasmin; Lane 3-300 microliters of prourokinase with methionine at position l58 and serine at position l60; Lane 4-300 microliters of prourokinase with methionine at position l58 and serine at position l60 previously treated with ll microliters (0.0l5 mg/ml) plasmin; Lane 5-standard molecular weight markers.

Table 6 shows the activation of glu-plasminogen (o) and lys-plasminogen (●) by glu-l58 prourokinase.

Table 7 shows the extent of clot lysis observed with each of three rabbits treated with either prourokinase or with a prourokinase derivative containing a mutant prourokinase gene.

## DESCRIPTION OF PREFERRED EMBODIMENTS

The prourokinase to be modified in accordance with this invention may be any prourokinase which is a plasminogen activator and has a single polypeptide chain form with an amino acid cleavage site for forming two chain urokinase. The cleavage site can occur at the lysine[158] - isoleucine[159] peptide bond. The prourokinase can be of molecular weight of about 50,000. The single chain species has been found to have the highest fibrinolytic activity and thus can act to dissolve fibrin clots without causing bleeding when introduced into the body of man and animals. Single polypeptide chain prourokinase as used in this specification, can have the following structure:

```
                              ┌──────S────────S──────┐
_____._____│                      │_____._____
ser    lys lys pro   cys   arg   phe lys ile ile   cys  ↑  leu
                                 157 158 159 160         │
                                                    active site
```

and is known in the art and further defined in UK Patent Application 2l2l050 "Preparation of Functional Human Urokinase Proteins" Heyneker, H.L., Holmes, W. E. and Vehar, G. A. and shown in Table I.

The above structure often breaks down into two chains on standing for relatively short periods of time in aqueous solution or when in the presence of such materials as plasminogen or other proteases when in the body where the lysine[158] - isoleucine[159] peptide bond cleaves as shown below:

```
                              ┌──────S────────S──────┐
_____._____│                      │_____._____
ser    lys lys pro   cys   arg   phe lys ile ile . cys  ↑  leu
                                 157 158 159 160         │
                                                    active site
```

It is the stability against such breakdown as illustrated in the latter structure which is the feature of the present invention, i.e., against cleavage at the lysine[158] - isoleucine[159] peptide bond.

Prourokinase is defined as that plasminogen activator having a single polypeptide chain and which exhibits plasminogen activator activity expressed in terms of CTA (Committee on Thrombolytic Agents) units as determined by a fibrin plate assay as described by Brakman, P. (1967) in Fibrinolysis, Schethema Holkima, Amsterdam, 1-124 and substantially as shown in Table I. Prourokinase in accordance with the preferred embodiment of this invention is human prourokinase, but other mammalian urokinase such as pig, house, dog and the like can be used and the term is meant to include all plasminogen activators which are in single chain form having fibrinolytic activity and capable of cleaving to the two chain form.

The preferred prourokinase to be modified in accordance with this invention is derived using recombinant DNA techniques from mRNA of mammalian kidney cells. The present invention can be practiced on any prourokinase derived using recombinant DNA techniques whose cleavage site from single chain to two chain occurs at a known amino acid cleavage site. Such single chain plasminogen activator materials which can be modified by this invention include such materials as described in Kohno, T., Hopper, P., Lillquist, J. S., Suddith, R. L., Greenlee, R. and Moir, D. T. (1984) Biotech. 2: 628-634; Husain, S. S., Gurewich, V. and Lipinski, B. (1983) Arch. Biochem. Biophys. 220: 31-38; Sumi, H., Kosugi, T., Matsuo, O. and Mihara, H. (1982) Acta Haematol. Japan. 45: 119-128; Collen, D., Stassen, J. M., Blaber, M., Winkler, M. and Verstraste, M. (1984) Thromb. Haemostas. 51: 2311-2314; and Wun, T.-C., Ossowski, L. and Reich, E. (1982) J. Biol. Chem. 257: 7262-7268; as well as the materials described in United States Patent 4,370,417 and UK Patent Application 2121050 "Preparation of Functional Human Urokinase Proteins" Heyneker, H. L., Holmes, W. E. and Vehar, G. A.

In the specific example of this invention, prourokinase is obtained from human kidney cells (Kohno, et al. (1984) supra). The cells are grown to confluency in Dulbecco's Modified Eagles medium supplemented with 5% heat-inactivated NuSerum (Collaborative Research, Inc., Lexington, Massachusetts). Confluent cells are harvested by centrifugation after treatment with 0.25 percent trypsin for 15 minutes at 35°C and are frozen in liquid nitrogen.

The poly (A) RNA is isolated according to the method of Maniatis, T., Fritsch, E. F. and Sambrook, J. (1982) "Molecular Cloning. A Laboratory Manual", Cold Spring Harbor Laboratory, Cold Spring Harbor, New York, 191-198. Briefly, 5 grams of frozen cells are lysed with NP-40 and the nuclei are removed by ultra-centrifugation. The cytoplasmic fraction is treated with proteinase K and the protein is removed by repeated phenol/chloroform:isoamyl alcohol extractions. The total cytoplasmic RNA is recovered by precipitation with ethanol with a yield of about 16 milligrams. The poly (A) RNA is isolated by oligo (dT) cellulose chromatography with a yield of about 200 micrograms. The intactness of the isolated poly (A) RNA is verified by in vitro translation in a rabbit reticulocyte lysate system

Conversion of poly (A) mRNA to double stranded cDNA is by standard procedures (see Wickens, M. P., Buell, G. N. and Schmidke, R. T. (1978) J. Biol. Chem. 253: 2483-2495) using oligo (dT) as the primer the first strand by reverse transcriptase, DNA polymerase I to synthesize the second strand, nuclease SI treatment to insure that the ends are flush and chromatography over Biogel Al5uM for size selection.

The size-selected double-stranded cDNA is ligated to HindIII synthetic oligonucleotide linkers (Collaborative Research, Inc., Lexington, Massachusetts) and then is transformed into a single-stranded fl phage vector (Zinder, N. D. and Boeke, J. D. (1982) Gene 19: 1-10; Bowden, D. W., Mao, J., Gill, T., Hsiao, K. Lillquist, J. S., Testa, D. and Vovis, G. F. (1984) Gene 27: 87-99).

Fifteen oligonucleotide probes, each 14 bases in length and homologous to portions of the PUK coding region, are synthesized by the automated phosphoramidite procedure (Applied Biosystems automated DNA synthesizer). The probes (number 1 to 15) are shown schematically in Table 2 together with their location within the gene.

For screening of the phage cDNA library, probes are mixed, labeled with $^{32}$P by means of polynucleotide kinase and gamma-$^{32}$P-ATP and isolated from the residual label by means of a polyacrylamide gel. The resultant labeled probes are used to probe nitrocellulose plaque lifts from the library by the method of Benton, W. D. and Davis, R. W. (1977) Science 196: 180-182. The use of multiple probes insures that unexpected occasional polymorphisms within the gene or poor hybridization by certain probes due to unpredictable structure or sequence context problems does not prevent identification of the clones containing PUK. In this manner, approximately 50,000 clones are screened for prourokinase sequences. One of the colonies shows hybridization with eleven of the probes. Dideoxy-sequencing and restriction analysis of the latter clone shows that the clone contains a 2.2 kilobase insert and starts in the middle of the signal sequence (SS) (see Table I). Further screening using a probe complimentary to a region of the signal sequence reveals a second clone which harbors the missing part of the signal sequence.

The two inserts are subcloned into an expression vector at a BglII restriction site which is common to both inserts. Restriction analysis is carried out on the resultant subclone and the latter cDNA is shown to be full length prourokinase.

Table 3 depicts the introduction of the gene encoding prourokinase into the eukaryotic expression vector pSV2 which is capable of replication and expression in mouse NIH 3T3 cells. Restriction endonuclease analysis of the plasmid DNA verifies that the correct construction is made. The secretion of PUK from mouse NIH 3T3 cells is verified by analysis on fibrin plate (Brakman (1967) supra) and by assaying for amidolytic activity (Kohno et al. (1984) supra).

In order to construct a plasmid containing PUK cDNA with an amino acid other than lysine at position 158, the scheme of Table 4 is used. Briefly, the plasmid pSV2 is cleaved with BglII then with EcoRI to generate a 0.23 kilobase BglII-EcoRI fragment containing lysine[158] and a 7.I kilobase BglII-EcoRI fragment The 0.23 kilobase fragment is verified by gel electrophoresis and gel eluted. The 0.23 kilobase BglII-EcoRI fragment is cleaved with DdeI to generate a 0.20 kilobase BglII-DdeI fragment which is verified by gel electrophoresis.

The 0.20 kilobase BglII-DdeI fragment is ligated in a three molecule reaction with the 7.I kilobase BglII-EcoRI fragment, and a synthetic oligomer, 30 oligucleotides in length (synthesized as described previously), complimentary to the section of the 0.20 kilobase BglII-DdeI fragment containing alanine at the lysine I58 position. (The three molecule ligation is repeated with synthetic oligomers containing either methionine, glutamic acid or valine at the lysine I58 position or methionine at the lysine I58 position and serine at position I60.) The fragments are ligated for 5 hours at I5° C and then a cell transformation is carried out. The correct ligation of the fragments creates a SacII restriction site as well as a new NruI restriction site. Restriction endonuclease analysis of one transformant verifies the creation of new SacII and NruII sites such that lysine[158] is replaced with an alanine residue. Dideoxy sequencing through the nucleotide region confirms replacement of the lysine at position I58 with alanine. Other transformants are picked and similarly verified to show replacement of the lysine at position I58 with methionine, glutamic acid, valine or methionine at position I58 and serine at position I60.

In order to obtain sufficient quantities of the above described prourokinase derivatives containing mutant prourokinase genes for biochemical characterization and in vivo studies, the expression plasmids carrying the lysine I58 mutant genes are co-transfected with a selective marker into mammalian cell cultures by the calcium precipitation procedure (Axel, R. and Wigler, M. H. United States Patent No. 4,399,2I6). The Chinese hamster ovary (CHO) strain designated DG44, which completely lacks the diploid dihydrofolate reductase (DHFR) locus (Urlaub, G., Kas, E., Carothers, A. M. and Chasin, L. A. (I983) Cell 33: 405-4I2), is used as host for expression. The plasmid designated pdhfr2.9, which carries a recombinant DNA engineered mouse DHFR minigene (Crouse, G. F., McEwan, R. N. and Pearson, M. L. (I983) Mol. Cell. Biol. 3: 257-266) is used as the selective vector for cotransfection.

Upon co-transfection, DHFR + colonies are obtained by selection in the growth medium depleted of glycine, hypoxanthine or guanine. The colonies are picked and expanded into standard cell culture T-flasks and their conditioned medis are assayed using an ELISA assay procedure. The PUK antibody used in the ELISA is rabbit polyclonal antibody and cross reacts with the mutated proteins. Thus, individual colonies which express the mutant proteins are identified by positive results with the ELISA.

In order to increase the production level of the protein being made, the above strains are then subjected to a gene amplification scheme (Kaufman, R. J. and Sharp, J. (I982) J. Mol. Biol. I59 60I-62I, Ringold, G., Dieckmann, B. and Lee, F. (I98I) J. Mol. App. Genetics I: i65-I75). The gene amplification procedure involved challenging cells with increasing concentrations of methotrexate (MTX), a specific inhibitor of DHFR. In response to MTX, the DHFR gene undergoes amplification and the gene copy number increases to hundreds to even thousands. Since co-transfection usually results in co-integration, the mutant PUK genes are co-amplified with the newly introduced DHFR gene. Routinely, the sequential increases of MTX concentrations are I0 nM , 50 nM, u.2 uM, I uM, 5 uM and 20 uM. However, in the course of experimentation, the amplification procedure is terminated when the production level is sufficient as judged by the ELISA. A yield of about 2 to I0 mg per liter is obtained after the step of 50 nM MTX challenge. A level of at least 50 mg per liter is obtained if the entire amplification procedure is followed.

## EXAMPLE I

Conditioned medium (3.9 liters) from amplified CHO ceils producing a mutant form of prourokinase (e.g., methionine substituted for lysine at position I58 and serine substituted for isoleucine at position I60) is filtered through a 0.2 uM pleated capsule filter (Gelman) to remove particulate material. The filtered conditioned medium is diluted to 7.4 liters with distilled water and then titrated to pH 7.0 with hydrochloric acid. The conductivity of the resulting solution is 5.0 mS/cm.

A 3.2 cm × I0.8 cm (87 ml) column of CM-TSK (Pierce Chemical Co. (Rockford, Illinois)) column is poured and equilibrated with 500 ml of 50 mM potassium phosphate, pH 7.0. The diluted and pH adjusted conditioned medium (7.4 liters) is applied to the CM-TSK column at a flow rate of 6.5 ml per minute. The column is then washed with 400 milliliters of 50 mM potassium phosphate pH 7.0 and then eluted with 400 ml of 50 mM potassium phosphate, 0.I M potassium chloride, pH 7.0 followed by 400 ml of 50 mM potassium phosphate, 0.I5 M potassium cholirde pH 7.0. Ten milliliter fractions are collected during the elution of the column and the protein content of each is determined by measuring the absorbance at 280 nm spectrophometrically. Fractions in the 50 mM potassium phosphate, 0.I5 M potassium chloride pH 7.0 eluate which contain material which absorbed light at 280 nm are pooled and are stored frozen at -20 degrees Celsius. The volume of the filtered CM-TSK pool is about 220 ml.

A I cm × 6.4 cm (5 ml) column of p-aminobenzamidine sepharose is poured and equilibrated with I00 ml of 50 mM sodium phosphate, I M sodium chloride, pH 7.5. The CM-TSK pool is thawed and the salt concentration adjusted by adding 37 ml of 5 M sodium chloride. The CM-TSK pool is then titrated to pH 2.2 using 2 N hydrochloric acid to dissolve particulate material, and then titrated to pH 7.5 with I N sodium hydroxide. The CM-TSK pool is filtered through a 0.2 micron filter (Millex GV, Millipore, Bedford, MA) and then applied to the p-aminobenzamidine column at a rate of I ml per minute. The column is then washed with 40 ml of 50 mM sodium phosphate, I M sodium chloride, pH 7.4 and then eluted with 80 ml of 0.I M sodium acetate, 0.I M sodium chloride, pH 4.8. Two milliliter fractions are collected during the elution of the column and the protein content of each is determined by measuring the absorbance at 280 nm spectrophometrically. Fractions with an absorbance at 280 nm greater than 0.02 are pooled. The resulting p-aminobenzamidine pool (44 ml) are stored frozen at -20 degrees Celsius.

Purity of the p-aminobenzamidine pool is determined by SDS-polyacryiamide gel electrophoresis (Laemmli, V.K., Nature (1970) 227, 680). Samples of the p-aminobenzamidine pool are run on a 5% acrylamide gel containing 0.1% SDS and analyzed by silver staining (Heukeshoven, J. and Dernick, R., Electrophoresis 6, 103-112, 1985). The molecular weight of the protein band in the p-aminobenzamidine pool is estimated to be 50,000 daltons. Protein content of the p-aminobenzamidine pool, determined by the method of Lowry, (Lowry, O.H., Rosenbrough, N.J., Farr, A.L., and Randt, R.J., J. Biol. Chem. 193, 265-275, 1951) is 0.027 mg/ml. The p-aminobenzamidine pool is concentrated by ultrafiltration. The final concentration of the concentrated p-aminobenzamidine pool is 0.19 mg/ml.

Sensitivity of the mutant prourokinase molecule to cleavage by plasmin is tested by incubating aliquots of the purified mutant prourokinase in the presence and absence of catalytic amounts of plasmin. An aliquot of the mutant prourokinase (i.e., the concentrated p-aminobenzamidine pool, 100 ul) and aliquot of a solution of prourokinase (100 ul of a 0.21 mg/ml solution), are dialyzed against 0.1% (v/v) acetic acid for two hours. After the dialysis an aliquot of each dialyzed solution containing 10 ug of protein is dried down using a vacuum centrifuge. The dried down mutant prourokinase and prourokinase samples are then dissolved in 0.9 m of 50 mM Tris-HCl, pH 7.4, 0.15 M sodium chloride, 0.1% Tween 80. These materials are used to prepare the four reaction mixtures: Reaction mixture 1 contained 30 microliters of prourokinase; Reaction mixture 2 contained 300 microliters of prourokinase and 11 microliters of plasmin (0.015 mg/ml human plasmin, Green Cross Corporation, Japan); Reaction mixture 3 contained 300 microliters of mutant prourokinase; and Reaction mixture 4 contained 300 microliters of mutant prourokinase and 11 microliters of plasmin (0.015 mg/ml human plasmin, Green Cross Corporation, Japan). The four reaction mixtures are incubated for three hours at 37 degrees Celsius. After incubation, 7.5 microliters of a 0.2 mg/ml solution of aprotinin are added to reactions 2 and 4. The reaction mixtures are then dialyzed against 0.1% (v/v) acetic acid for two hours. After the dialysis each dialyzed solution is dried down using a vacuum centrifuge. The samples are then each dissolved in 50 microliters of 50 mM Tris-HCl, pH 6.8, 2% sodium dodecyl sulfate, 20% glycerol, and 10 mM dithiothreitol and then subjected to electrophoresis according to the method of Laemmli (Laemmli, V.K., Nature 227, 680, 1970). After electrophoresis proteins in the gel are visualized by silver staining (Heukeshoven, J. and Dernick, R., Electrophoresis 6, 103-112, 1985). A photograph of the gel is shown in Table 5. The gel shows that under these conditions prourokinase is completely degraded by incubation with plasmin whereas the mutant prourokinase is not degraded by incubation with plasmin.

## EXAMPLE 2

Conditioned medium from amplified CHO cells producing a mutant form of prourokinase (a glutamic acid substituted for the lysine at amino acid position 158) is filtered through a 0.2 uM pleated capsule filter (Gelman) to remove particulate material. The filtered conditioned medium is titrated to pH 7.0 with phosphoric acid and then diluted with 13.0 liters of distilled water. The conductivity of the resulting solution is 5.2 mS/cm.

A 4.4 cm × 15.2 cm (200 ml) column of CM-TSK (Pierce Chemical Co.) column is poured and equilibrated with 500 ml of 50 mM sodium phosphate, pH 7.0. The dilluted and pH adjusted conditioned medium (19.2 liters) is applied to the CM-TSK column at-a flow rate of 16 ml per minute. The column is then washed with 810 milliliters of 50 mM sodium phosphate pH 7.0 and then eluted with 600 ml of 50 mM sodium phosphate, 0.1 M sodium chloride, pH 7.0 followed by 600 ml of 50 mM sodium phosphate, 0.15 M sodium chloride pH 7.0. Ten milliliter fractions are collected during the elution of the column and the protein content of each is determined by measuring the absorbance at 280 nm spectrophometrically. Fractions from the trailing portion of the protein peak in the 50 mM sodium phosphate, 0.1 M sodium chloride, pH 7.0 eluate are pooled and are stored frozen at -20 degrees Celsius. The volume of the filtered CM-TSK pool is about 130 ml.

A 1 cm × 10.2 cm (8 ml) column of p-aminobenzamidine sepharose is poured and equilibrated with 100 ml of 50 mM sodium phosphate, 1 M sodium chloride, pH 7.5. The CM-TSK pool is thawed and filtered through a 0.2 micron filter (Millex GV, Millipore, Bedford, MA). Sodium chloride (7.5 g) is dissolved in the filtered CM-TSK pool. The pool is then applied to the p-aminobenzamidine column at a rate of 1 ml per minute. The column is then washed with 25 ml of 50 mM sodium phosphate, 1 M sodium chloride, pH 7.4 and then eluted with 60 ml of 0.1 M sodium acetate, 0.1 M sodium chloride, pH 4.8. Two milliliter fractions are collected during the elution of the column and the protein content of each is determined by measuring the absorbance at 280 nm spectrophometrically. Fractions with an absorbance at 280 nm greater than 0.037 are pooled. the resulting p-aminobenzamidine pool (31 ml) is stored frozen at -20 degrees Celsius.

Purity of the p-aminobenzamidine pool is determined by SDS-polyacrylamide gel electrophoresis as described above. Eighty-six microliters of the p-aminobenzamidine pool is run on a 12% acrylamide gel containing 0.1% SDS and analyzed by silver staining (Heukeshoven, J. and Dernick, R., Electrophoresis 6, 103-112, 1985). The molecular weight of the protein band in the p-aminobenzamidine pool is estimated to be 50,000 daltons. Protein content of the p-aminobenzamidine pool, determined by the method of Lowry, (Lowry, O.H., Rosenbrough, N.J., Farr, A.L., and Randt, R.J., J. Biol. Chem. 193, 265-275, 195) is 0.035 mg/ml.

The ability of the purified Glu158 mutant PUK to activate plasminogen is tested using an assay described by Collen et al. (Collen, D., Zamarron, C., Lijnen, H.R., and Hoylaerts, J. Biol. Chem. 261: 1259-1266, 1986). In this assay PUK (or mutant PUK) is incubated with plasminogen in the presence of an excess amount of the chromogenic substrate for plasmin (D-Val-Leu-Lys-pNA). Monitoring the hydrolysis of the chromogenic substrate (i.e., the accumulation of pNA chromophore which absorbs light at 405nm) is a means of following the sum of the following two reactions:

```
1.    Plasminogen ----------------------------> Plasmin
                 Mutant Plasminogen Activator

2.    D-Val-Leu-Lys-pNA ---------> D-Val-Leu-Lys + pNA (chromophor)
                         Plasmin
```

Monitoring the instantaneous rate of hydroysis of the chromogenic substrate can be used to quantitate the amount of plasmin (i.e., the result of plasminogen activation) generated in the reaction. The rate of plasminogen activation is monitored by plotting the slope of the absorbance curve as a function of time.

Two 500 microliter reaction mixtures are prepared by mixing reagents prewarmed to 37 degrees Celsius as described below: Reaction mixture I is made by mixing I29.5 microliters of 50 mM Tris-HCl, 38 mM sodium chloride, 0.I% Tween 80, pH 7.4, and 44.5 microliters of Glu-plasminogen (I mg/ml, Kabivitrum) and 326 microliters of a solution containing I0.5 microliters of the p-aminobenzamidine pool, 488 microliters of 50 mM Tris-HCl, 38 mM sodium chloride, 0.I% Tween 80, pH 7.4 and 503 microliters of a 3.5 mM stock solution of D-Val-Leu-Lys-pNA (S-225I, KabiVitrum) made up in 50 mM Tris-HCl, 38 mM sodium chloride, 0.I% Tween 80, pH 7.4. Reaction mixture 2 is made by mixing I55 microliters of 50 mM Tris-HCl, 38 mM sodium chloride, 0.I% Tween 80, pH 7.4, and I9 microliters of Lys-plasminogen (2 mg/ml, American Diagnostica, Grenich, Connecticut) and 326 microliters of a solution containing I0.5 microliters of the p-aminobenzamidine pool, 488 microliters of 50 mM Tris-HCl, 38 mM sodium chloride, 0.I% Tween 80, pH 7.4 and 503 microliters of a 3.5 mM stock solution of D-Val-Leu-Lys-pNA (S-225I, KabiVitrum, Stockholm, Sweden) made up in 50 mM Tris-HCl, 38 mM sodium chloride, 0.I% Tween 80, pH 7.4.

Reaction mixture I and reaction mixture 2 are pipetted into cuvettes prewarmed to 37 degrees Celsius. The cuvettes are then loaded into a spectrophotometer (Beckman DU-6). The absorbance of the reaction mixtures and the change in absorbance per minute is monitored at 405 nm at 20 second intervals for 20 minutes. The absorbance of the reaction mixtures and the change in absorbance per minute at I, 5, I0, I5, and 20 minutes are plotted as a function of time and are shown in Table 6.

EXAMPLE 3

Three prourokinase derivatives are purified for in vivo thrombolytic testing: The first derivative (Met/Ser) had a methionine substituted for the lysine which is normally present at amino acid position I58 and a serine substituted for the isoleucine normally present at amino acid position I60. The second derivative (Glu) had a Glutamic acid substituted for the lysine which is normally present at amino acid position I58. The third prourokinase derivative (Ala) had an Alanine substituted for the lysine which is normally present at amino acid position I58. These prourokinase derivatives are purified essentially as described below.

Purification of the Met/Ser Prourokinase Derivative. Conditioned medium (3.9 liters) from amplified CHO cells producing the Met/Ser prourokinase derivative is filtered through a 0.2 uM pleated capsule filter (Gelman) to remove particulate material. The filtered conditioned medium is diluted to 7.4 liters with distilled water and then titrated to pH 7.0 with hydrochloric acid. The conductivity of the resulting solution is 5.0 mS/cm. The diluted and pH adjusted conditioned medium (7,4 liters) is applied to a 3.2 cm × I0.8 cm (87 ml) column of CM-TSK (Pierce Chemical Co.) equilibrated with 50 mM potassium phosphate, pH 7.0 at a flow rate of 6.5 ml per minute. The column is then washed with 400 milliliters of 50 mM potassium phosphate pH 7.0 and then eluted with 400 ml of 50 mM potassium phosphate, 0.I M potassium chloride, pH 7.0 foliowed by 400 ml of 50 mM potassium phosphate, 0.I5 M potassium cholirde pH 7.0. Ten milliliter fractions are collected during the elution of the column and the protein content of each is determined by measuring the absorbance at 280 nm spectrophometrically. Fractions in the 50 mM potassium phosphate, 0.I M potassium chloride pH 7.0 eluate which contained material which absorbed light at 280 nm are pooled and are stored frozen at -20 degrees Celsius. The CM-TSK pool (240 ml) is thawed, titrated to pH 2.2 using 2 N hydrochloric acid to dissolve particulate material, and then titrated to pH 7.5 with I N sodium hydroxide. Fifty milliliters of 5 M sodium chloride is added to the CM-TSK pool which is then filtered through a 0.2 micron filter (Millex GV, Millipore, Bedford, MA) and then appied to a 2.5 cm × 5.I cm (25 ml) column of p-aminobenzamidine sepharose (Collaborative Research, Inc., Bedford, MA), equilibrated with I00 ml of 50 mM sodium phosphate, I M sodium chloride, pH 7.5 at a rate of I ml per minute. The column is then washed with 50 mM sodium phosphate, IM sodium chloride, pH 7.4 and then eluted with 0.I M sodium acetate, 0.I M sodium chloride, pH 4.8. Five milliliter fractions are collected during the elution of the column and the protein content of each is determined by measuring the absorbance at 280 nm spectrophometrically. Fractions with an absorbance at 280 nm greater than 0.07 are pooled. The resulting p-aminobenzamidine pool (40 ml) is stored frozen at -20 degrees Celsius. Protein content of the p-aminobenzamidine pool, determined by the method of Lowry, (Lowry, O.H., Rosenbrough, N.J., Farr, A.L., and Randt, R.J., (I95I) J. Biol. Chem. 193, 265-275) is 0.26 mg/ml.

Purification of the Glu Prourokinase Derivative. Conditioned medium (3.6 liters) from amplified CHO cells producing the Glu prourokinase derivative is filtered through a 0.2 uM pleated capsule filter (Gelman) to remove particulate material. The filtered conditioned medium is diluted to l0.8 liters with distilled water and then titrated to pH 7.0 with hydrochloric acid. The conductivity of the resulting solution is 5.6 mS/cm. The diluted and pH adjusted conditioned medium (l0.8 liters) is applied to a 4.4 cm × l0.8 cm (l64 ml) column of CM-TSK (Pierce Chemical Co.) equilibrated with 50 mM potassium phosphate, pH 7.0 at a flow rate of 7.0 ml per minute. The column is then washed with 500 milliliters of 50 mM potassium phosphate pH 7.0 and then eluted with 50 mM potassium phosphate, 0.l M potassium chloride, pH 7.0 followed by 50 mM potassium phosphate, 0.l5 M potassium cholirde pH 7.0. Fourteen milliliter fractions are collected during the elution of the column and the protein of each is determined by measuring the absorbance at 280 nm spectrophometrically. The four protein peaks in the 50 mM potassium phosphate, 0.l M sodium chloride pH 7.0 eluate are pooled and are stored frozen at -20 degrees Celsius. The total volume of the four pools is 340 ml. Purity is assessed by SDS-gel electrophoresis (Laemmli, V.K., Nature). Pools which are judged to be less 90% pure are further purified by reverse phase HPLC on a C4 column as described below. The CM-TSK pools are thawed and filtered through a 0.2 micron filter (Millex GV, Millipore, Bedford, MA). Each CM-TSK pool is adjusted to 0.l% Trifluoroacetic acid and is then individually applied to a C4 reverse phase column equilibrated with l5% propanol, 0.l% trifluoroacetic acid at a rate of l ml/ minute. The Glu derivative is eluted with a propanol gradient (l5% - 50%, in 0.l% Trifluoroacetic acid). The major peak eluting from the column with a retention time of about 25 minutes is collected and dialyzed against 0.l% v/v acetic acid for l6 hours at 4 degrees Celsius. After dialysis the samples are frozen at -20 degrees Celsius. The pools from two such CM-TSK purifications are thawed and combined and concentrated by ultrafiltration. The protein concentration of the concentrated Glu derivative (36 ml) is determined by the method of Lowry (Lowry, O.H., Rosenbrough, N.J., Farr, A.L., and Randt, R.J., (l95l) J. Biol. Chem. 193, 265-275) to be 0.22 mg/ml. Purification of the Ala Prourokinase Derivative. Conditioned medium (2.0 liters) from amplified CHO cells producing the Ala prourokinase derivative is filtered through a 0.2 uM pleated capsule filter (Gelman) to remove particulate material. The filtered conditioned medium is diluted to 5.6 liters with distilled water and then titrated to pH 7.0 with phosphoric acid. The conductivity of the resulting solution is 5.4 mS/cm. The diluted and pH adjusted conditioned medium (5.6 liters) is applied to a 3.2 cm × ll.5 cm (92 ml) column of CM-TSK (Pierce Chemical Co.) equilibrated with 50 mM sodium phosphate, pH 7.0 at a flow rate of 6.0 ml per minute. The column is then washed with l liter of 50 mM sodium phosphate pH 7.0 and then eluted with of 50 mM sodium phosphate, 0.l5 M sodium chloride, pH 7.0. Five milliliter fractions are collected during the elution of the column and the protein content of each is determined by measuring the absorbance at 280 nm spectrophometrically. The final peak of protein eluting from the column is pooled and is stored frozen at -20 degrees Celsius. The CM-TSK pool (70 ml) is thawed, adjusted to l M sodium chloride by the addition of 3.47 grams of solid sodium chloride and applied to a l.5 cm × 5.0 cm column of p-aminobenzamidine sepharose (Collaborative Research, Inc., Bedford, MA), equilibrated with l00 ml of 50 mM sodium phosphate, l M sodium chloride, pH 7.5 at a rate of l ml per minute. The column is then washed with three column volumes of 50 mM sodium phosphate, l M sodium chloride, pH 7.5 and then eluted with four column volumes (36 ml) of 0.l M sodium acetate, u.l M sodium chloride, pH 4.8. The resulting p-aminobenzamidine pool is stored frozen at -20 degrees Celsius. Protein content of the p-aminobenzamidine pool, determined by the method of Lowry is 0.3l mg/ml.

The purified Met/Ser, Glu, and Ala derivatives of prourokinase are compared to prourokinase in their ability to facilitate clot dissolution (i.e., thrombolysis) in vivo using the rabbit jugular vein thrombolysis model system (Collen, et al., J. Clin. Invest. 7l, 368-376, l983; Collen, D, Stassen, J.M., Wimkler, M., and Verstraete, M., Thromb. Haemostas. 52, 27-30, l984). Prourokinase is obtained from Collaborative Research, Inc., Bedford, MA. For the in vivo studies, rabbits (2.5-3 kg) with artificially produced fibrin clots present in their jugular vein are infused with prourokinase or with one of the prourokinase derivatives at a dose of l mg/kg over a period of four hours. Thirty minutes after the infusion is completed, extent of clot lysis is determined. Three rabbits are tested for each of the derivatives and for prourokinase. The data for the experiment (i.e., extent of clot lysis observed with each rabbit treated with either prourokinase derivative, are shown in Table 7.

As described above, the lysine at position l58 in the prourokinase is removed and substituted with one or more amino acids alone or in conjunction with amino acid substitute at position l60. Such amino acids include the nonpolar (hydrophobic) class comprising alanine, leucine, isoleucine, valine, proline, phenylalanine, tryptophan and methionine; the polar neutral class of glycine, serine, threonine, cysteine, tyrosine, asparagine and glutamine; the positively charged (basic) class comprising arginine, lysine and histidine; and the negatively charged (acidic) class containing aspartic acid and glutamic acid. Similarly, the isoleucine at position l57 either alone or in combination with lysine l58 can be removed and substituted with one or more amino acids in order to increase stability.

The substitution for the lysine l58 alone provides a single chain prourokinase which is amino acid modified and is more resistant to conversion to the two chain form than is the case with naturally occurring single chain prourokinase; however, in some cases stability may be increased to the point where no cleavage occurs at the former lysine site under normal body conditions and other conditions of use in clot dissolution. Cleavage of the normally occurring prourokinase can occur rapidly in the body and in storage .

The starting single chain prourokinase and the resulting amino acid modified prourokinase have a high fibrin selectivity. High fibrin selectivity is described in Zamarron et al. (l984) supra, Sumi, H., Toki, N., Sasaki, K. and Mihara, H. (l983) in Progress in Fibrinolysis (Davidson, J. F., Bachmann, F., Bouvier, C. A. and Kruithof, E. K. O.,

eds., Churchhill Livingstone, Edinburgh) 6: l65-l67 and Collen et al. (l984) supra.

The modification of the lysine amino acid can be carried out by genetic recombinant means as for example by splicing into the DNA an oligomer which contains the desired amino acid change, or by hybridizing and priming the DNA with an oligomer containing the changed amino acid such that synthesis with DNA polymerase and subsequent cloning of the synthesized DNA would result in a "mutant" DNA containing the desired amino acid change.

In some cases, rather than using genetic modification techniques to remove or replace the lysine, chemical treatment can be carried out as for example by altering the side-chain of the desired amino acid by alkylation with reagents such as n-ethylmaleimide, dinitroflourobenzene and 2-methoxy-5-nitrotropone or by acylation with n-carboxyanhydrides, diketene and methyl acetimidate.

In those plasminogen activators where the two chain form is obtained by cleavage of the single chain form at sites other than lysine - isoleucine, suitable modification of the cleavage site in accordance with this invention can be used to stabilized such activators. For example other amino acid substitutions can be used.

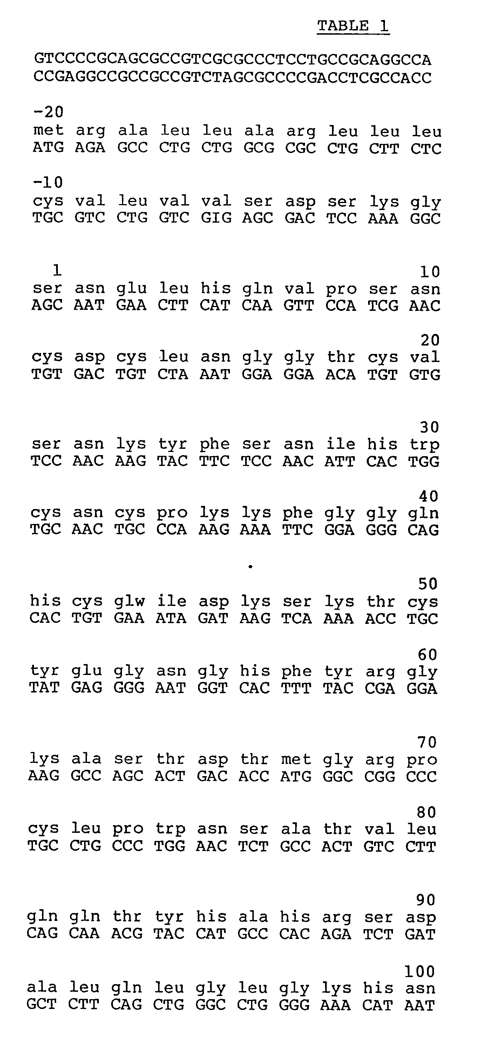

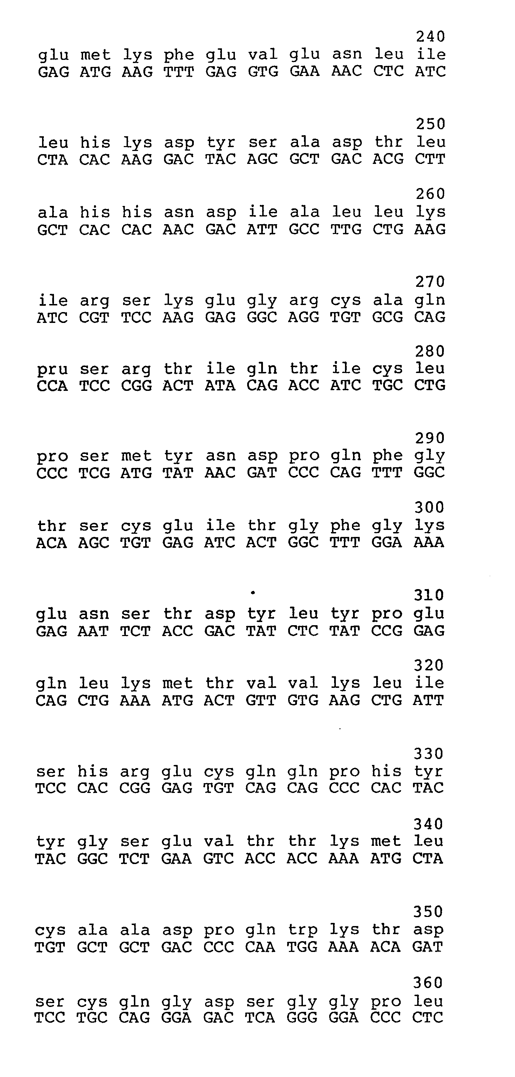

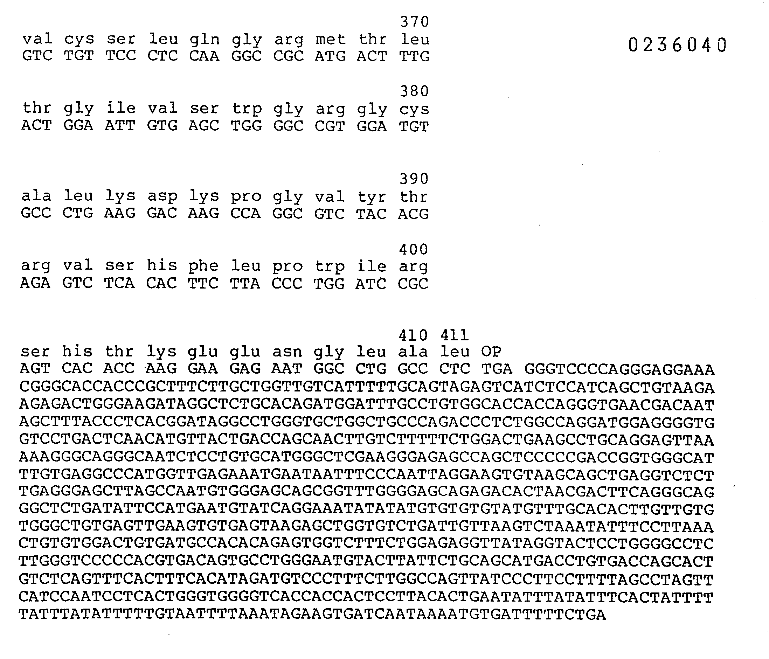

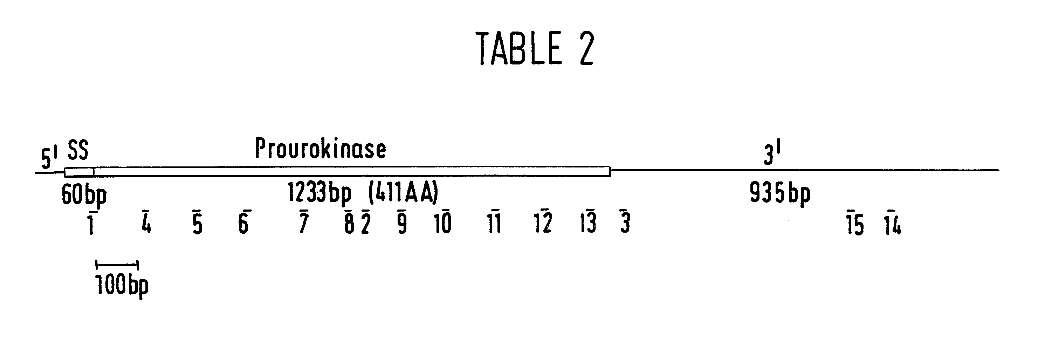

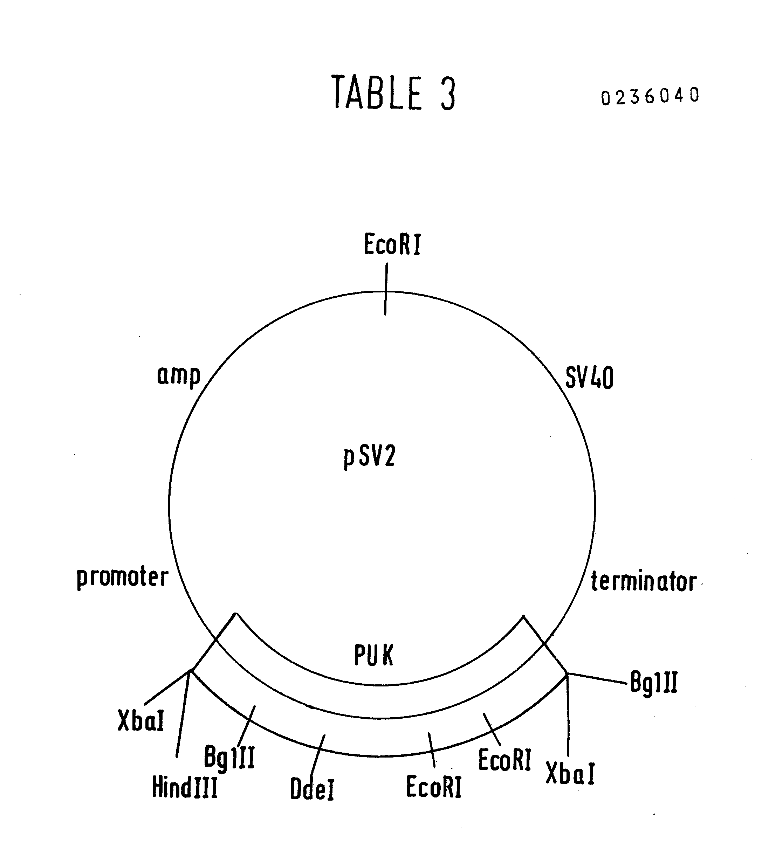

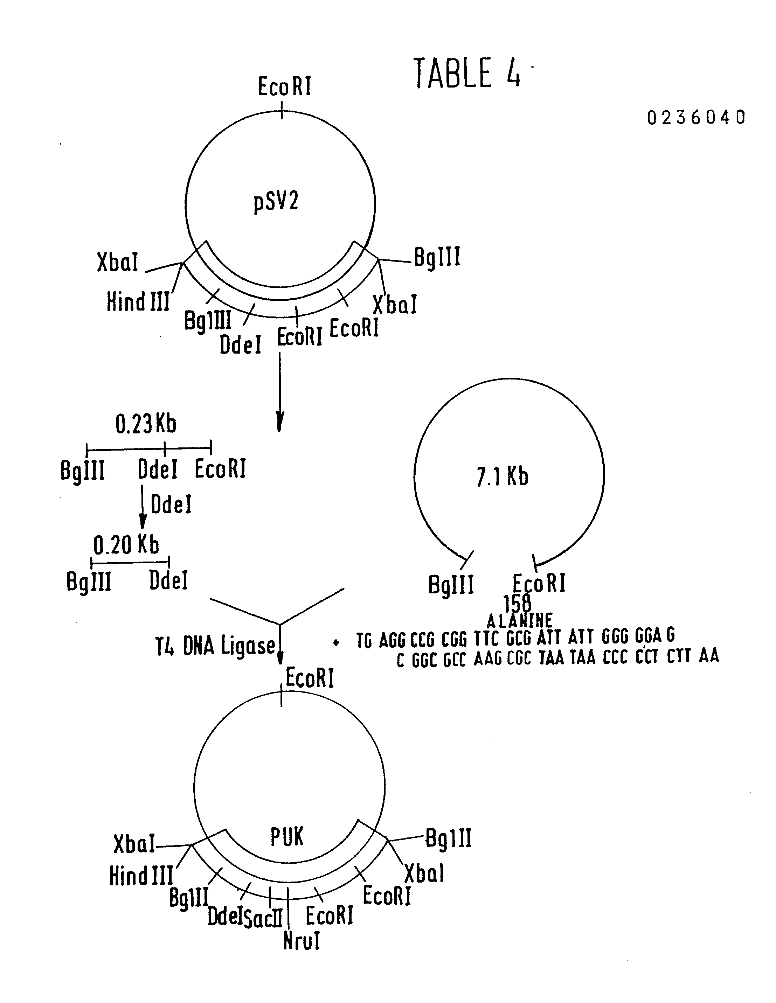

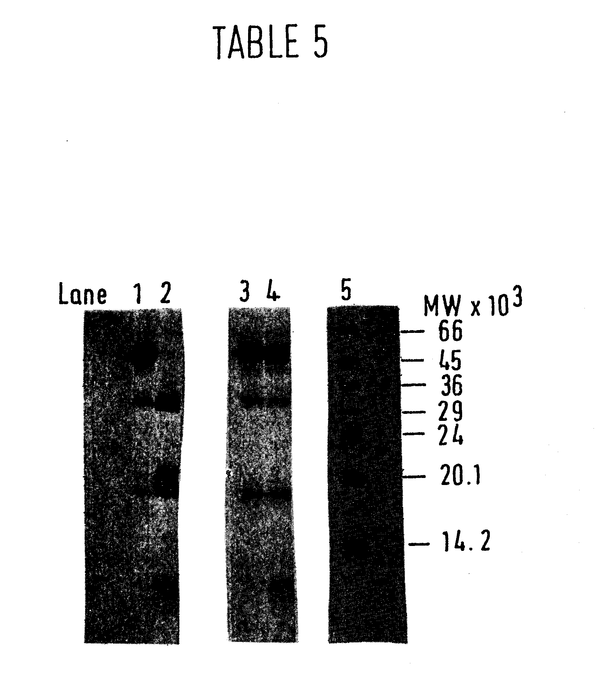

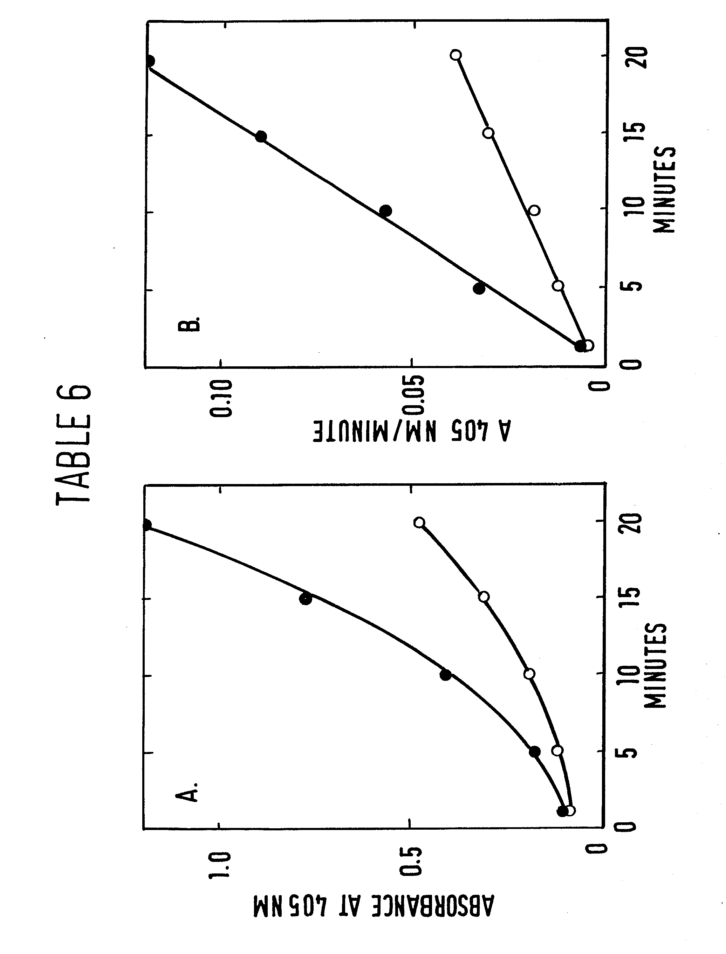

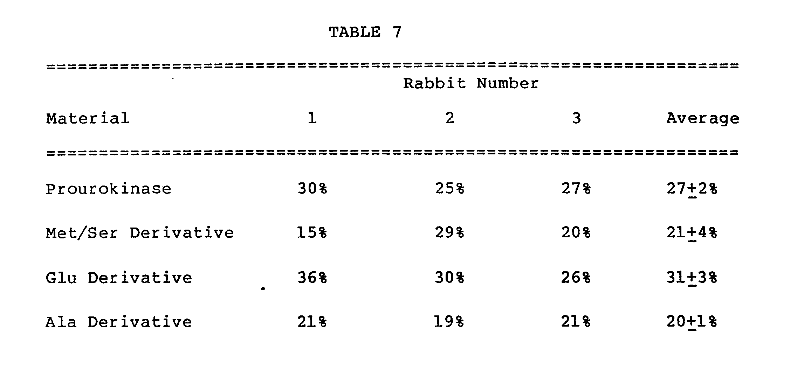

## Claims

l. An amino acid modified form of prourokinase having a single polypeptide chain, high fibrin selectivity and resistant to cleavage to the two-chain form to an extent greater than its original unmodified form.

2. A method of increasing the resistance to cleavage of singe chain prourokinase without substantially decreasing the fibrinolytic activity, said method comprising treating prourokinase so as to modify the lysine residue at position l58 by substituting an amino acid at said site.

3. The method of Claim 2 further comprising genetically engineering the prourokinase and replacing the lysine[158] by site-directed mutagenesis.

4. The method of Claim 2 further comprising replacing the lysine[158] by chemical modification.

5. A method of increasing the resistance to cleavage of a single chain plasminogen activator without substantially decreasing the fibrinolytic activity,

said method comprising genetically engineering said plasminogen activator to replace a cleavage site thereon,

said genetic engineering comprising,

removing a first DNA fragment containing a lysine[158]-isoleucine[159] cleavage site,

introducing into the first removed DNA fragment site a second fragment having a modified cleavage site.

6. A method in accordance with the method of claim 5 wherein said first removed DNA fragment site is replaced with a second DNA fragment which is the same as the first DNA fragment except that it has a modified cleavage site.

7. A method in accordance with the method of claim 6 wherein said second DNA fragment has an amino acid substitution at position l58 such that the substituted amino acid is an amino acid other than lysine.

8. A method in accordance with the method of claim 7 wherein the first DNA fragment is removed with restriction endonuclease treatment.

9. A method in accordance with the method of claims 7 wherein said second DNA fragment is introduced by ligation with T4 DNA ligase.

l0. A structurally modified single chain plasminogen activator having a lysine cleavage site removed by substituting an alternate amino acid at said cleavage site to resist change to the two chain form.

ll. A structurally modified single chain prourokinase in accordance with Table I having its lysine-isoleucine cleavage site removed and replaced with another amino acid.

l2. A structurally modified single chain prourokinase in accordance with claim ll wherein said amino acid is selected from the group consisting of methionine and alanine.

TABLE 1

GTCCCCGCAGCGCCGTCGCGCCCTCCTGCCGCAGGCCA
CCGAGGCCGCCGCCGTCTAGCGCCCCGACCTCGCCACC

-20
met arg ala leu leu ala arg leu leu leu
ATG AGA GCC CTG CTG GCG CGC CTG CTT CTC

-10
cys val leu val val ser asp ser lys gly
TGC GTC CTG GTC GIG AGC GAC TCC AAA GGC

 1                                    10
ser asn glu leu his gln val pro ser asn
AGC AAT GAA CTT CAT CAA GTT CCA TCG AAC

                                      20
cys asp cys leu asn gly gly thr cys val
TGT GAC TGT CTA AAT GGA GGA ACA TGT GTG

                                      30
ser asn lys tyr phe ser asn ile his trp
TCC AAC AAG TAC TTC TCC AAC ATT CAC TGG

                                      40
cys asn cys pro lys lys phe gly gly gln
TGC AAC TGC CCA AAG AAA TTC GGA GGG CAG

                 •
                                      50
his cys glw ile asp lys ser lys thr cys
CAC TGT GAA ATA GAT AAG TCA AAA ACC TGC

                                      60
tyr glu gly asn gly his phe tyr arg gly
TAT GAG GGG AAT GGT CAC TTT TAC CGA GGA

                                      70
lys ala ser thr asp thr met gly arg pro
AAG GCC AGC ACT GAC ACC ATG GGC CGG CCC

                                      80
cys leu pro trp asn ser ala thr val leu
TGC CTG CCC TGG AAC TCT GCC ACT GTC CTT

                                      90
gln gln thr tyr his ala his arg ser asp
CAG CAA ACG TAC CAT GCC CAC AGA TCT GAT

                                     100
ala leu gln leu gly leu gly lys his asn
GCT CTT CAG CTG GGC CTG GGG AAA CAT AAT

```
                                    240
glu met lys phe glu val glu asn leu ile
GAG ATG AAG TTT GAG GTG GAA AAC CTC ATC


                                    250
leu his lys asp tyr ser ala asp thr leu
CTA CAC AAG GAC TAC AGC GCT GAC ACG CTT

                                    260
ala his his asn asp ile ala leu leu lys
GCT CAC CAC AAC GAC ATT GCC TTG CTG AAG


                                    270
ile arg ser lys glu gly arg cys ala gln
ATC CGT TCC AAG GAG GGC AGG TGT GCG CAG

                                    280
pru ser arg thr ile gln thr ile cys leu
CCA TCC CGG ACT ATA CAG ACC ATC TGC CTG


                                    290
pro ser met tyr asn asp pro gln phe gly
CCC TCG ATG TAT AAC GAT CCC CAG TTT GGC

                                    300
thr ser cys glu ile thr gly phe gly lys
ACA AGC TGT GAG ATC ACT GGC TTT GGA AAA


                 .                  310
glu asn ser thr asp tyr leu tyr pro glu
GAG AAT TCT ACC GAC TAT CTC TAT CCG GAG

                                    320
gln leu lys met thr val val lys leu ile
CAG CTG AAA ATG ACT GTT GTG AAG CTG ATT


                                    330
ser his arg glu cys gln gln pro his tyr
TCC CAC CGG GAG TGT CAG CAG CCC CAC TAC

                                    340
tyr gly ser glu val thr thr lys met leu
TAC GGC TCT GAA GTC ACC ACC AAA ATG CTA


                                    350
cys ala ala asp pro gln trp lys thr asp
TGT GCT GCT GAC CCC CAA TGG AAA ACA GAT

                                    360
ser cys gln gly asp ser gly gly pro leu
TCC TGC CAG GGA GAC TCA GGG GGA CCC CTC
```

0236040

```
                                        370
val cys ser leu gln gly arg met thr leu
GTC TGT TCC CTC CAA GGC CGC ATG ACT TTG


                                        380
thr gly ile val ser trp gly arg gly cys
ACT GGA ATT GTG AGC TGG GGC CGT GGA TGT


                                        390
ala leu lys asp lys pro gly val tyr thr
GCC CTG AAG GAC AAG CCA GGC GTC TAC ACG


                                        400
arg val ser his phe leu pro trp ile arg
AGA GTC TCA CAC TTC TTA CCC TGG ATC CGC


                                        410 411
ser his thr lys glu glu asn gly leu ala leu OP
AGT CAC ACC AAG GAA GAG AAT GGC CTG GCC CTC TGA GGGTCCCCAGGGAGGAAA
CGGGCACCACCCGCTTTCTTGCTGGTTGTCATTTTTGCAGTAGAGTCATCTCCATCAGCTGTAAGA
AGAGACTGGGAAGATAGGCTCTGCACAGATGGATTTGCCTGTGGCACCACCAGGGTGAACGACAAT
AGCTTTACCCTCACGGATAGGCCTGGGTGCTGGCTGCCCAGACCCTCTGGCCAGGATGGAGGGGTG
GTCCTGACTCAACATGTTACTGACCAGCAACTTGTCTTTTTCTGGACTGAAGCCTGCAGGAGTTAA
AAAGGGCAGGGCAATCTCCTGTGCATGGGCTCGAAGGGAGAGCCAGCTCCCCCGACCGGTGGGCAT
TTGTGAGGCCCATGGTTGAGAAATGAATAATTTCCCAATTAGGAAGTGTAAGCAGCTGAGGTCTCT
TGAGGGAGCTTAGCCAATGTGGGAGCAGCGGTTTGGGGAGCAGAGACACTAACGACTTCAGGGCAG
GGCTCTGATATTCCATGAATGTATCAGGAAATATATATGTGTGTGTATGTTTGCACACTTGTTGTG
TGGGCTGTGAGTTGAAGTGTGAGTAAGAGCTGGTGTCTGATTGTTAAGTCTAAATATTTCCTTAAA
CTGTGTGGACTGTGATGCCACACAGAGTGGTCTTTCTGGAGAGGTTATAGGTACTCCTGGGGCCTC
TTGGGTCCCCCACGTGACAGTGCCTGGGAATGTACTTATTCTGCAGCATGACCTGTGACCAGCACT
GTCTCAGTTTCACTTTCACATAGATGTCCCTTTCTTGGCCAGTTATCCCTTCCTTTTAGCCTAGTT
CATCCAATCCTCACTGGGTGGGGTCACCACCACTCCTTACACTGAATATTTATATTTCACTATTTT
TATTTATATTTTTGTAATTTTAAATAGAAGTGATCAATAAAATGTGATTTTTCTGA
```

# TABLE 2

5' SS          Prourokinase          3'

60 bp          1233 bp   (411 AA)          935 bp

1    4    5    6    7    8 2    9    10    11    12    13    3          15   14

100 bp

# TABLE 3

EcoRI

amp

SV40

pSV2

promoter

terminator

PUK

XbaI

BglII

HindIII

BglII

DdeI

EcoRI

EcoRI

XbaI

TABLE 4

0236040

EcoRI

pSV2

XbaI          BgIII
Hind III                    XbaI
BglIII                EcoRI
DdeI    EcoRI EcoRI

0.23 Kb

BgIII    DdeI    EcoRI

DdeI

0.20 Kb

BgIII    DdeI

7.1 Kb

BgIII        EcoRI
158
ALANINE
• TG AGG CCG CGG TTC GCG ATT ATT GGG GGA G
C GGC GCC AAG CGC TAA TAA CCC CCT CTT AA

T4 DNA Ligase

EcoRI

PUK

XbaI                        BgIII
Hind III                        XbaI
BgIII                EcoRI
DdeI SacII    EcoRI
NruI

# TABLE 5

Lane 1 2 3 4 5 MW x 10³

— 66
— 45
— 36
— 29
— 24
— 20.1
— 14.2

# TABLE 6

0236040

TABLE 7

| Material | Rabbit Number | | | |
|---|---|---|---|---|
| | 1 | 2 | 3 | Average |
| Prourokinase | 30% | 25% | 27% | 27+2% |
| Met/Ser Derivative | 15% | 29% | 20% | 21+4% |
| Glu Derivative | 36% | 30% | 26% | 31+3% |
| Ala Derivative | 21% | 19% | 21% | 20+1% |